# NOUVEAU FASCICULE DE BREVET EUROPEEN

(11) **EP 0 107 559 B2**
(45) Date de publication et mention de la décision concernant l'opposition: **17.12.1997**
(45) Mention de la délivrance du brevet: 24.06.1987
(21) Numéro de dépôt: 83401954.9
(22) Date de dépôt: 06.10.1983
(51) Int. Cl.: A61K 9/50, B01F 5/06

(54) **Procédé d'homogénéisation de dispersions de phases lamellaires lipidiques hydratées**
Verfahren zur Homogenisierung von Dispersionen von hydrierten lipidischen lamellaren Phasen
Process for homogenising dispersions of hydrated lipidic lamellar phases

(30) Priorité: 15.10.1982 FR 8217311
(43) Date de publication de la demande: 02.05.1984
(73) Titulaire: PARFUMS CHRISTIAN DIOR, 75008 Paris (FR)
(72) Inventeur: Redziniak, Gérard, F-78500 Sartrouville (FR); Meybeck, Alain, F-92400 Courbevoie (FR)
(74) Mandataire: Durand, Yves Armand Louis

(56) Documents cités:
- EP-A- 0 004 223
- EP-A- 0 021 337
- EP-A- 0 036 676
- EP-A- 0 041 772
- CH-A- 366 518
- DE-A- 2 630 586
- DE-A- 2 938 807
- US-A- 3 873 720
- US-A- 4 016 100
- US-A- 4 280 996
- US-A- 4 411 894
- "HAGERS HANDBUCH DER PHARMAZEUTISCHEN PRAXIS", edition 4, vol. 7, partie A, page 448, 1971, Springer-Verlag, Berlin, DE
- Emulsion: Theory and Practice, P. Becher, 1st ed. Rheinhold publ. USA, pp. 226-239, 1958
- Ann. Biophys. Bioeng., 1980, 9, pp. 467-508
- Febs Letters, vol. 99, no. 1, 1979, pp. 210-214
- Trends in Pharmacological Sciences, vol. 3, no 7, 1982, pp. 274-277
- Journal of Lipid Research, vol. 21, 1980, pp. 981-992
- W.J. Lecluse "Homogénéisateurs à haute pression", Informations chimie no 191, 1979, pp. 1-8 (suppl.)
- Liposomes- A practical Approach, RRC New, page 137, IRL Press
- Pharmazie in unser Zeit, 1982, no 4, pp. 97-102
- Biochim. et Biophys. Acta, 557, 1979, pp. 9-23
- Chemical Engineering, May 13, 1973, pp. 87-92
- Galenica (5) Les Systemes dispersés, I: Agents de surface et émulsions, Puisieux et Seiller, Paris, 1983
- Les cosmetiques "pharmacolotie et biologie", 1962, pp. 91-97
- Ann. Pharmaceutiques francaises, 1983, 41, no1, pp. 3-13
- Parfums, cosmétiques et aromes, no 36, 1980, pp. 73-79
- Seifen-Oele-Fette-Wachse, 116 Jg, 14/1990, pages 509-515

## Description

La présente invention concerne un procédé pour l'homogénéisation de dispersions de phases lamellaires lipidiques hydratées telles que, par exemple, des liposomes, et a plus particulièrement pour objet un procédé d'homogénéisation sous pression d'une telle dispersion.

On connaît déjà plusieurs procédés tels que la dialyse, les ultrasons, des procédés mécaniques, permettant d'homogénéiser une suspension de phases lamellaires hydratées pour obtenir une suspension de phases lamellaires telles que des liposomes, relativement homogène. Par exemple, les documents EP-A 4467 ou EP-A-36 676 révèlent un procédé consistant à effectuer une homogénéisation de liposomes mécaniquement dispersés par passage dans un filtre du type nucléopore, suivi d'une dialyse pour enlever le médicament libéré des vésicules. Les vésicules contenant le médicament sont ensuite recueillies pour être ensuite extrudées sous pression élevée au travers d'un orifice de faible dimension (voir page 22 de EP-4467 en particulier).

Toutefois, ces procédés permettent de traiter à chaque opération seulement de petites quantités de suspension, en particulier en nécessitant la préparation de liposomes par le procédé de dépôt d'une couche lipidique à l'intérieur d'un évaporateur rotatif comme décrit dans EP-4467 page 21, et en détruisant les liposomes lors du traitement d'homogénéisation comme il ressort de ce qui précède. Ils sont donc difficilement exploitables pour la fabrication industrielle de suspensions de liposomes.

La présente invention a pour but de remédier à tous ces inconvénients en proposant un nouveau procédé d'homogénéisation d'une dispersion de phases lamellaires lipidiques hydratées permettant d'homogénéiser un volume de suspension de phases lamellaires lipidiques hydratées acceptable d'un point de vue industriel. De plus, la qualité de l'homégénéisation ainsi réalisée permet d'éviter l'addition d'émulsionnants et l'utilisation de conservateurs étant donné que la suspension homogène peut subir une filtration stérilisante, par exemple.

Il permet d'obtenir, de façon tout à fait inattendue, un taux d'encapsulation très élevé dans lesdites phases lamellaires de substances éventuellement présentes dans la phase aqueuse de ladite suspension.

A cet effet, la présente invention a pour objet un procédé pour l'obtention d'une suspension homogénéisée de liposomes dans un liquide aqueux contenant une substance, consistant à disperser des constituants lipidiques contenant un ou plusieurs lipides amphiphiles dans ledit liquide aqueux, puis à homogénéiser la dispersion de liposomes obtenue en alimentant cette dernière à une pression comprise entre 10000 et 70000 kPa dans un passage de faible largeur défini entre les parois d'un orifice et les bords d'un élément obturateur disposé de manière réglable dans la section dudit orifice, à l'encontre du flux de ladite dispersion, pour ainsi réaliser l'élévation du taux d'encapsulation de ladite substance dans les liposomes.

Selon une autre caractéristique du procédé, la pression d'alimentation est comprise entre 10 000 kPa et 40 000 kPa et peut être égale à 30 000 kPa environ.

Selon encore une autre caractéristique du procédé, la phase lamellaire lipidique est une phase lamellaire lipidique peu hydratée ayant une teneur en phase lipidique entre 50% et 80%.

Suivant une autre caractéristique du procédé selon l'invention, on réalise plusieurs passages successifs de la dispersion dans le passage précité, avec éventuellement un refroidissement de ladite dispersion entre chaque passage.

En outre, selon un mode de réalisation avantageux, la dispersion précitée contient de 50% à 99,9% de liquide et de 50 à 0,1% de la phase lipidique, ou de 20 à 99,9% de liquide et de 80 à 0,1% de la phase lamellaire lipidique hydratée précitée.

Le liquide précité est, de préférence, une solution aqueuse physiologique de chlorure de sodium ou de d-glucose, le liquide étant éventuellement une solution d'un produit actif à encapsuler dans la phase lipidique.

Selon une autre caractéristique du procédé de l'invention, la suspension à homogénéiser est alimentée sous pression sous une atmosphère inerte.

L'invention a également pour objet une suspension de phases lamellaires lipidiques hydratées obtenue par le procédé décrit ci-dessus dont les particules ont une dimension moyenne comprise entre 30 et 150 nanomètres et en moyenne de 100 nanomètres environ, les dimensions ont été évaluées au moyen de la microscopie électronique et leur moyenne à l'aide d'un appareil dit: 〈〈Nano-Sizer〉〉.

Avantageusement, cette suspension est stérilisée par une filtration stérilisante, de préférence à la température ambiante. Ainsi, il n'est pas nécessaire d'additionner à la suspension homogénéisée obtenue par le procédé de l'invention des additifs de conservation souvent indésirables dans les produits à usage pharmaceutique ou cosmétique.

L'invention sera mieux comprise et d'autres caractéristiques, détails et avantages de celle-ci apparaîtront plus clairement au cours de la description explicative qui va suivre faite en référence aux dessins schématiques annexés donnés uniquement à titre d'exemple, et dans lesquels:
La figure 1 est un schéma illustrant le principe du procédé d'homogénéisation de l'invention.
La figure 2 est un schéma synoptique illustrant une installation pour la production d'une suspension homogénéisée selon le procédé de l'invention.
La figure 3 est un diagramme représentant la variation des dimensions des particules contenues dans la suspension en fonction de la durée de traitement de l'homogénéisation; et
La figure 4 est un diagramme dont la courbe (c) montre la variation de la différence de dimension entre les particules avant le traitement d'homogénéisation et après le traitement d'homogénéisation en fonction de la pression d'alimentation de la suspension et la courbe (d) indique les variations du taux d'encapsulation du produit actif dans la phase lamellaire lipidique hydratée en fonction de la pression d'homogénéisation.

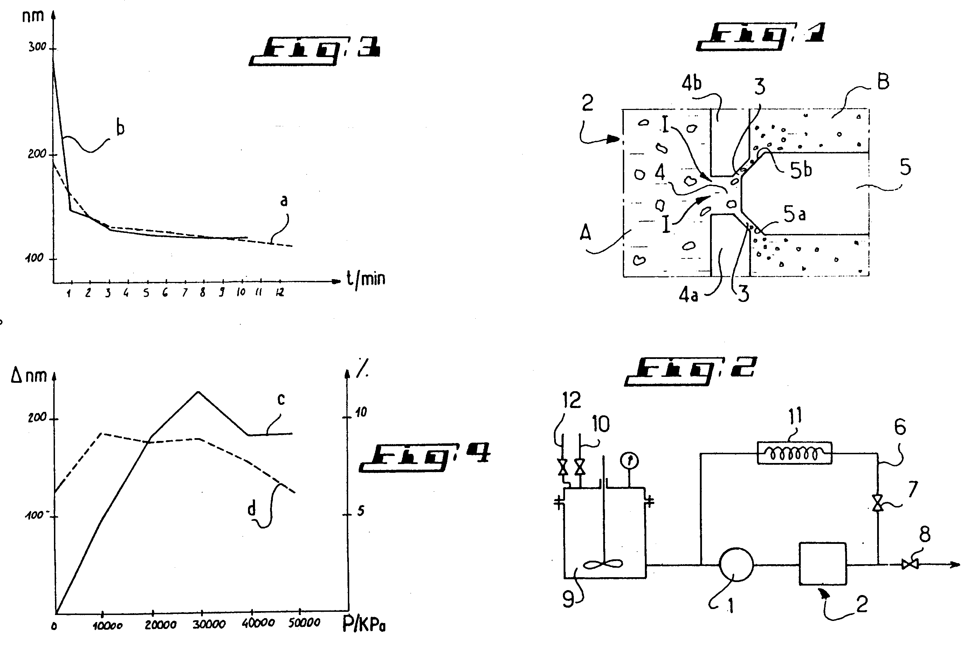

En se référant aux figures 1 et 2, le procédé de l'invention pour l'obtention de suspensions homogénéisées de phases lamellaires lipidiques hydratées dans le liquide telles que, par exemple, une suspension de liposomes, consiste tout d'abord à disperser une phase lipidique ou une phase lamellaire lipidique contenant un ou plusieurs lipides amphiphiles tels que par exemple de la lécithine dans un liquide pour obtenir une dispersion de grosses particules représentées symboliquement par la phase A de la figure 1.

Le liquide est de préférence une solution aqueuse physiologique de chlorure de sodium dans laquelle est éventuellement dissous un produit biologiquement actif et/ou ayant des propriétés organoleptiques.

En outre la phase lipidique qui peut contenir un ou plusieurs lipides amphiphiles choisis par exemple parmi les composés appartenant à la famille des glycolipides, phospholipides ou phospho-aminolipides, tels que, par exemple, la lécithine de soja ou d'oeuf, peut également contenir des composés à caractère hydrophobe tels que par exemple le cholestérol ou l'ubiquinone.

Cette phase lipidique peut être obtenue par tout procédé susceptible de former des phases lamellaires, et par exemple par le procédé d'atomisation décrit dans la demande de brevet européen EP-A-0 087 993.

Par ailleurs, la phase lamellaire lipidique dispersée dans le liquide peut être une phase lamellaire lipidique très hydratée ou une phase lamellaire lipidique peu hydratée comme cela sera illustré ci-après dans les exemples, dans laquelle a été éventuellement encapsulé un produit biologiquement actif et/ou possédant des propriétés organoleptiques.

La dispersion ainsi obtenue est alimentée sous pression au moyen d'une pompe 1 dans un appareil 2 dont le principe est illustrè à la figure 1 et comprenant un passage 3 de faible largeur défini entre les parois 4a, 4b d'un orifice 4 et les bords 5a, 5b d'un élément obturateur 5 qui est disposé de manière réglable dans la section de l'orifice 4, à l'encontre du flux de la dispersion A représentée par la flèche I. Sous l'effet de la pression et des turbulences provoquées par les faibles dimensions du passage 3, les particules contenues dans la suspension sont réduites en de fines particules de dimensions de l'ordre de quelques dizaines de nanomètres à quelques centaines de nanomètres.

Dans un mode de réalisation préféré du procédé de l'invention appliqué dans l'installation illustré à la figure 2, la dispersion est réalisée dans le bac à dispersion 9 au moyen, par exemple, d'une agitation mécanique, la dispersion A ainsi obtenue est aspirée par la pompe 1 puis introduite sous pression dans l'appareil 2. A la sortie de l'appareil, la suspension homogénéisée B obtenue est soit soutirée pour son utilisation soit en partie ou totalement recyclée en amont de la pompe 1 pour un nouveau passage dans l'appareil 2 par le conduit 6 et au moyen des vannes 7, 8. Avantageusement, pour éviter un échauffement de la suspension par ces compressions successives et ces passages dans l'appareil 2, on refroidit la suspension B recyclée au moyen d'un réfrigérateur 11.

Avantageusement, l'alimentation de la suspension A dans la pompe 1 peut être réalisée sous pression à partir d'un réservoir d'alimentation appelé réservoir de pré-alimentation qui peut être constitué soit par le bac de dispersion 9 soit par un autre récipient disposé entre le bac de dispersion 9 et la pompe 1. Dans l'exemple illustré, nous considérons que le bac de dispersion 9 constitue également le réservoir de pré-alimentation sous pression.

Pour effectuer cette pré-alimentation sous pression, le bac de dispersion est rendu étanche aux gaz et on alimente au-dessus de la dispersion A un gaz sous pression, avantageusement un gaz inerte tel que de l'azote, ou un gaz rare, sous une pression comprise entre environ 100 kPa et 1000 kPa pour avoir une atmosphère non oxydante de préférence, par le conduit d'alimentation 10. Cette pré-alimentation sous pression est fortement souhaitée lorsque la dispersion A a une viscosité élevée par exemple de l'ordre de 1 à 10 Pa.s et notamment pour l'homogénéisation d'une suspension de phases lamellaires lipidiques peu hydratées, ayant une teneur en phase lipidique proche de 50% environ. De plus, l'alimentation du liquide de dispersion et de la phase lipidique sous forme de poudre ou de la phase lamellaire lipidique à disperser peut être réalisée en continu dans le bac de dispersion 9 par la conduite 12, par exemple.

Le débit qui peut être traité selon le procédé de l'invention est relativement important et fonction notamment de la viscosité de la dispersion à homogénéiser et de la pression d'alimentation dans l'appareil 2. A titre d'exemple, sous une alimentation de 35 000 kPa, le débit de suspension traitée par le procédé de l'invention est égal à environ 57 litres par heure.

Par ailleurs, les avantages du procédé d'homogénéisation sous pression de l'invention seront clairement illustrés par les exemples suivants de fabrication de suspension homogénéisée de phases lamellaires lipidiques hydratées, ces exemples, à l'exception de l'exemple 1 qui ne fait pas partie de l'invention, en ce qu'il n'illustre qu'un procédé d'atomisation ne doivent en aucun cas être considérés comme limitant le cadre de la présente invention étant donné que de nombreuses variations et modifications notamment dans les produits utilisés sont possibles.

### Exemple 1

production d'un mélange pulvérulent de constituants lipidiques par atomisation: à 20 g de lécithine de soja on ajoute 0,3 g d'ubiquinone et 120 millilitres de chloroforme. Ce mélange est atomisé à 75°C selon le procédé de la demande de brevet européen EP-A-0 087 993. Après 15 mn on obtient 18 g de poudre jaune sans odeur particulière avec un rendement de 90%.

### Exemple 2

a) production d'une poudre par atomisation. Dans un bécher de 100 millilitres on introduit 5 g de lécithine de soja solubilisées dans 25 millilitres de chloroforme et 0,05 g de dopamine dissoute dans 10 millilitres de méthanol.
   La solution obtenue est atomisée selon le procédé de l'exemple 1 à 75°C et on recueille une poudre blanche.
b) obtention d'une dispersion. La poudre recueillie en (a) est introduite dans un bécher de 1 litre contenant 5 g de polypeptides de collagène en solution dans 500 millilitres de solution aqueuse de chlorure de sodium à 0,9%. Le mélange est soumis à une agitation magnétique pendant 2 heures à température ambiante.
   A la fin de l'agitation, la taille moyenne des liposomes mesurée au nano-sizer est de 254 nanomètres.
c) homogénéisation sous pression de la dispersion. On alimente la dispersion obtenue en (b) dans l'orifice 3 de l'appareil 2 sous une pression de 40 000 kPa et un débit de 50 litres par heure avec recyclage de la solution en amont de la pompe d'alimentation. L'homogénéisation est maintenue pendant 5 minutes et la taille moyenne des liposomes de la suspension est égale à 98 nanomètres.

### Exemple 3

Dans un bécher de 2 litres on introduit 10 g de poudre de lécithine de soja obtenue par atomisation, selon le procédé de l'exemple 1, 10 g de polypeptides de collagène et 920 g d'une solution physiologique de NaCl à 0,9%. On mélange pendant 15 minutes à température ambiante au moyen d'un agitateur à hélice.

La dispersion ainsi obtenue est ensuite traitée aux ultrasons à l'aide d'un sonificateur à lames vibrantes pendant 1 heure sous refroidissement. A la suite de ce traitement, la taille moyenne des liposomes passe de 300 nanomètres à 190 nanomètres.

Ensuite, on soumet la suspension obtenue après sonification à une homogénéisation sous pression conforme au procédé de l'invention en alimentant ladite suspension sous une pression de 35 000 kPa.

On détermine la taille moyenne des liposomes à différentes durées d'homogénéisation sous pression. Les résultats obtenus sont représentés par la courbe (a) de la figure 4 portant en ordonnées la taille moyenne des liposomes et en abscisses la durée d'homogénéisation à 35 000 kPa.

Ces résultats montrent que l'on obtient très rapidement des particules de dimension moyenne de l'ordre de 100 nanomètres ce qui est nettement inférieur aux dimensions obtenues par le procédé de sonification.

### Exemple 4

Dans un bécher de 1 litre on introduit 25 g de poudre de lécithine de soja obtenue par atomisation, selon le procédé de l'exemple 1, 2,5 g de sel disodique d'adénosine triphosphate (Na₂ATP) et 472,5 g d'une solution de NaCl à 0,9%. On mélange pendant 15 minutes à température ambiante au moyen d'un agitateur à hélice. On soumet la suspension ainsi obtenue au procédé d'homogénéisation sous pression de l'invention avec une pression d'alimentation de 40 000 kPa. La taille des liposomes ainsi obtenue est mesurée pour différentes durées de traitement. Les résultats obtenus sont représentés par la courbe (b) de la figure 3 qui montre comme dans l'exemple 3 que l'on obtient très rapidement des particules de taille moyenne d'environ 100 nanomètres.

D'après les exemples 3 et 4, on déduit donc que le procédé d'homogénéisation de l'invention permet d'obtenir une suspension ayant des particules de taille moyenne très petite même si l'on traite une dispersion contenant des particules de taille moyenne relativement élevée.

De plus, sur des dispersions préparées comme dans l'exemple 4, on effectue une homogénéisation sous pression pendant une durée déterminée de 5 minutes à différentes pressions d'alimentation. A chaque suspension obtenue on mesure la taille moyenne des particules ou liposomes ainsi que le taux d'encapsulation de Na₂ATP dans ces liposomes.

Les résultats obtenus sont représentés par les courbes (c) et (d) de la figure 4.

La courbe (c) représente la variation de la différence de taille moyenne des liposomes entre la taille avant homogénéisation sous pression et la taille après 5 minutes de traitement pour différentes pressions de travail. La diminution maximum de taille moyenne des particules est obtenue pour des pressions supérieures à environ 20 000 kPa.

La courbe (d) représente le pourcentage d'encapsulation de Na₂ATP obtenu après 5 minutes d'homogénéisation sous pression de la suspension de liposomes, à différentes pressions d'alimentation. Cette courbe montre que l'on obtient un taux d'encapsulation maximum pour des pressions d'alimentation comprise entre 10 000 kPa et 40 000 kPa environ.

En outre, il ressort de cette même figure 4, qu'à une pression de 30 000 kPa environ, la courbe (c) traduisant la finesse des liposomes après homogénéisation et la courbe (d) indiquant le taux d'encapsulation obtenu passent toutes deux par un maximum. Ceci montre l'intérêt, dans le procédé selon l'invention, de choisir des pressions de cet ordre de grandeur.

### Exemple 5

a) Préparation d'une phase lamellaire lipidique peu hydratée: On verse dans le bac de dispersion 9 de l'installation représentée à la figure 2, 3 kilogrammes d'une solution aqueuse, par exemple, une solution physiologiue de NaCl contenant un produit physiologiquement actif tel qu'un extrait d'aloès et 3 kilogrammes d'une poudre de lécithine obtenue par atomisation selon le procédé de l'exemple 1 qui peut contenir un constituant à caractère hydrophobe. On obtient ainsi une phase lamellaire lipidique peu hydratée.
b) production de la suspension homogénéisée: A la phase lamellaire lipidique peu hydratée on ajoute une solution aqueuse de dilution contenant par exemple 0,9% de NaCl ou 0,5% de d-glucose pour obtenir une dispersion contenant de 0,10 à 80% de la phase lamellaire lipidique peu hydratée, puis on alimente ce mélange dans le passage 3 de l'appareil 2 pour réaliser une homogénéisation sous pression. Le taux d'encapsulation de l'extrait d'aloès dans les liposomes ainsi produits est compris entre 50 et 70% environ.

Dans une variante, la phase lamellaire peu hydratée peut être préparée par dispersion de la poudre atomisée de lécithine contenant éventuellement un constituant à caractère hydrophobe dans une quantité inférieure d'une solution aqueuse à encapsuler. On peut ainsi obtenir une phase lamellaire lipidique peu hydratée contenant jusqu'à 80% de phase lipidique. Le mélange est réalisé par exemple au broyeur à rouleaux, et est ensuite dilué dans une solution aqueuse de dilution pour obtenir une dispersion contenant de 0,1 à 80% de ladite phase lamellaire lipidique peu hydratée comme indiqué ci-dessus. Le taux d'encapsulation de la solution à encapsuler est compris également entre 50 et 70%.

Les exemples ci-dessus montrent qu'il est possible avec le procédé d'homogénéisation de l'invention, d'obtenir des suspensions homogénéisées de liposomes ou de phases lamellaires lipidiques peu hydratées stables. Comme le volume de dispersion traitée par unité de temps est important et que le procédé peut être conduit en continu, il est donc possible de produire des quantités importantes de dispersion de phases lamellaires lipidiques compatibles avec une production industrielle.

Par ailleurs, comme la dimension des liposomes est de l'ordre de 100 nanomètres environ, il est possible de stériliser ces suspensions par filtration stérilisante comptye tenu que les microorganismes ou bactéries ont des dimensions supérieures à 220 nanomètres.

Ainsi, on peut stériliser à froid la suspension de liposomes ce qui est très important compte tenu que les composés constituant les liposomes ou encapsulés dans lesdits liposomes sont souvent dégradables par la température. De plus, comme la suspension est stérilisée, il n'est plus nécessaire d'ajouter des conservateurs.

Par ailleurs, comme la suspension obtenue est très homogène, il n'est généralement d'ajouter des émulsionnants pour stabiliser cette suspension.

Le procédé d'homogénéisation sous pression de l'invention, permet donc d'obtenir des suspensions de liposomes présentant une meilleure stabilité et homogénéité que celle obtenue par dialyse et permet surtout de produire notamment, de manière continue avec un taux d'encapsulation satisfaisant des quantités industrielles de dispersion ce qui n'est absolument pas possible avec le procédé d'homogénéisation aux ultrasons. De plus, l'homogénéisation sous pression peut être effectuée à froid sans élévation de température tandis que le procédé aux ultrasons demande un refroidissement de la solution pour éviter une dégradation des produits constituants les liposomes.

Toutefois, comme le montre la baisse du taux d'encapsulation observée dans l'exemple 4, l'alimentation de la dispersion sous une pression trop élevée peut aboutir à une détérioration des liposomes. En outre à pression trop élevée, on obtient un échauffement important des solutions (dispersons) qui est souvent préjudiciable.

## Revendications

1. Procédé pour l'obtention d'une suspension homogénéisée de liposomes dans un liquide aqueux contenant une substance, consistant à disperser des constituants lipidiques contenant un ou plusieurs lipides amphiphiles dans ledit liquide aqueux, puis à homogénéiser la dispersion de liposomes obtenue en alimentant cette dernière à une pression comprise entre 10000 et 70000 kPa dans un passage de faible largeur défini entre les parois d'un orifice et les bords d'un élément obturateur disposé de manière réglable dans la section dudit orifice, a l'encontre du flux de ladite dispersion, pour ainsi réaliser l'élévation du taux d'encapsulation de ladite substance dans les liposomes.

2. Procédé selon la revendication 1, caractérisé en ce que la pression d'alimentation est comprise entre 10 000 kPa et 40 000 kPa.

3. Procédé selon la revendication 1, caractérisé en ce que la pression d'alimentation précitée est de 30 000 kPa environ.

4. Procédé selon l'une des revendications 1 à 3, caractérisé en ce qu'on réalise plusieurs passages successifs de la dispersion dans le passage précité, avec éventuellement un refroidissement de ladite dispersion entre chaque passage.

5. Procédé selon l'une des revendications précédentes, caractérisé en ce que la dispersion à homogénéiser est pré-alimentée sous pression dans un dispositif effectuant l'alimentation sous pression de la dispersion dans le passage précité.

6. Procédé selon la revendication 5, caractérisé en ce que ladite pré-alimentation sous pression de la dispersion est réalisée sous atmosphère inerte ou non oxydante.

7. Procédé selon l'une des revendications précédentes, caractérisé en ce que le liquide de dispersion précité est une solution d'un produit biologiquement actif et/ou possédant des propriétés organoleptiques.

8. Procédé selon l'une des revendications 1 à 6, caractérisé en ce que le liquide précité de la dispersion est une solution aqueuse physiologique de chlorure de sodium ou de d-glucose.

## Claims

1. Method for the obtainment of a homogenized suspension of liposomes in an aqueous liquid containing a substance, consisting in dispersing lipidic components containing one or several amphiphilous lipids in the said aqueous liquid, then in homogenizing the dispersion of the liposomes obtained by feeding the latter at a pressure lying between 10,000 kPa and 70,000 kPa into a passageway of small width defined between the walls of an opening and the edges of a shutter element disposed in an adjustable manner in the section of the said opening, in opposition to the flow of the said dispersion, to thus achieve the increase of the rate of encapsulation of the said substance into the liposomes.

2. Method according to claim 1, characterized in that the feed pressure lies between 10,000 kPa and 40,000 kPa.

3. Method according to claim 1, characterized in that the aforesaid feed pressure is about 30,000 kPa.

4. Method according to one of claims 1 to 3, characterized in that one carries out several successive passages of the dispersion within the aforesaid passageway with possibly a cooling of the said dispersion between each passage.

5. Method according to one of the foregoing claims, characterized in that the dispersion to be homogenized is pre-fed under pressure into a device performing the feeding under pressure of the dispersion into the aforesaid passageway.

6. Method according to claim 7, characterized in that the said pre-feeding under pressure of the dispersion is carried out in an inert or non-oxidizing atmosphere.

7. Method according to one of the foregoing claims, characterized in that the aforesaid dispersion liquid is a solution of a product which is biologically active and/or has organoleptic properties.

8. Method according to one of claims 1 to 6, characterized in that the aforesaid liquid of the dispersion is an aqueous physiological solution of sodium chloride or d-glucose.

## Patentansprüche

1. Verfahren zur Erhaltung einer homogenisierten Aufschwemmung von Liposomen in einer eine Substanz enthaltenden wässerigen Flüßigkeit, welches darin besteht, ein oder mehrere amphiphile Lipide enthaltende lipidische Bestandteile in der besagten wässerigen Flüßigkeit zu dispergieren, dann die erhaltene Dispersion von Liposomen zu homogenisieren durch Einspeisung der letzteren unter einem zwischen 10000 kPa und 70000 kPa liegenden Druck in einen zwischen den Wänden einer Öffnung und den Rändern eines in dem Querschnitt der besagten Öffnung in einstellbarer Weise angeordneten Verschlußelementes abgegrenzten Durchgang schwacher Breite entgegen der Strömung der besagten Dispersion, um somit die Erhöhung des Einkapselungsverhältnisses der besagten Substanz in den Liposomen zu bewirken.

2. Verfahren nach Anspruch 1, dadurch gekennzeichnet, daß der Speisedruck zwischen 10000 kPa und 40000 kPa liegt.

3. Verfahren nach Anspruch 1, dadurch gekennzeichnet, daß der vorgenannte Speisedruck ungefähr 30000 kPa beträgt.

4. Verfahren nach einem der Ansprüche 1 bis 3, dadurch gekennzeichnet, daß man mehrere aufeinanderfolgende Durchströmungen der Dispersion innerhalb des vorgenannten Durchgangs durchführt, gegebenenfalls mit einer Abkühlung der besagten Dispersion zwischen jeder Durchströmung.

5. Verfahren nach einem der vorangehenden Ansprüche, dadurch gekennzeichnet, daß die zu homogenisierende Dispersion unter Druck in eine die Druckzufuhr der Dispersion in den vorgenannten Durchgang gewährleistende Vorrichtung voreingespeist wird.

6. Verfahren nach Anspruch 5, dadurch gekennzeichnet, daß die besagte Voreinspeisung der Dispersion unter Druck in einer inerten bzw. nicht oxidierenden Atmosphäre durchgeführt wird.

7. Verfahren nach einem der vorangehenden Ansprüche, dadurch gekennzeichnet, daß die vorgenannte Dispersionsflüßigkeit eine Lösung eines biologisch aktiven und/oder organoleptische Eigenschaften besitzenden Produktes ist.

8. Verfahren nach einem der Ansprüche 1 bis 6, dadurch gekennzeichnet, daß die vorgenannte Flüßigkeit der Dispersion eine wässerige physiologische Lösung von Natriumchlorid oder d-Glücose ist.
